# EUROPÄISCHE PATENTSCHRIFT

(11) **EP 0 336 220 B1**
(45) Veröffentlichungstag und Bekanntmachung des Hinweises auf die Patenterteilung: **26.01.1994**
(21) Anmeldenummer: 89105144.3
(22) Anmeldetag: 22.03.1989
(51) Int. Cl.: A61B 17/22, A61B 8/08, G10K 11/00

(54) **Wasserkreislauf eines Lithotripters**
Water circulation for a lithotripter
Circulation de l'eau pour un lithotripteur

(30) Priorität: 02.04.1988 DE 3811316
(43) Veröffentlichungstag der Anmeldung: 11.10.1989
(73) Patentinhaber: DORNIER MEDIZINTECHNIK GMBH, D-82101 Germering (DE)
(72) Erfinder: Erhardt, Wolfgang, Dipl.-Ing., D-8080 Fürstenfeldbruck (DE); Grözinger, Reiner, Dipl.-Ing., D-8031 Alling (DE); Hagelauer, Ulrich, Dr.Ing., D-8000 München 19 (DE); Maassen, Udo, D-8000 München 40 (DE); Windsheimer, Manfred, Dipl.-Ing., D-8034 Germering (DE)
(74) Vertreter: Zwergel, Wilhelm

(56) Entgegenhaltungen:
- EP-A- 090 138
- EP-A- 0 265 741
- DE-A- 2 745 347
- DE-A- 3 503 477
- DE-A- 3 536 073

## Beschreibung

Die Erfindung betrifft den Wasserkreislauf eines Geräts zur Stosswellenbehandlung, insbesondere eines Lithotripters.

Aus dem Prospekt: Vom Ende der Steinzeit, HV 5000 08/87 der DORNIER MEDIZINTECHNIK GMBH, 8034 Germering, ist ein Lithotripter bekannt, bei dem die Stosswellenapplikation mit einem Wasserkissen erfolgt, über das die Stosswelle in den Patientenkörper eingekoppelt wird. Für die verlustlose Erzeugung und Einkoppelung der Stosswelle wird im Wasserkissen als Koppelmedium entgastes und enthärtetes Wasser benutzt, das auf 37°C temperiert wird. Zwischen das Wasserkissen und den Patientenkörper wird ein Koppelgel appliziert, wie dies in bekannter Weise in der Ultraschall-Diagnostik durchgeführt wird. Zur blasenfreien Ankoppelung der beiden Grenzflächen Wasser/Patientenkörper und zum Ausgleich von Unebenheiten des Patientenkörpers, ist ein bestimmter Ankoppelungsdruck des Wasserkissens erforderlich. Für diese Niveau-Einstellung des Wasserkissens (Anpassung an verschieden korpulente Patienten) und die Konstanthaltung des Ankoppelungsdrucks wird eine Druckregelung für den Druck des Koppelmediums innerhalb des Wasserkissens benötigt. Durch den hohen Freiheitsgrad der Therapieeinheit - so ist ein Verschwenken über, seitlich und unter den Patientenkörper möglich - werden aufgrund der durch die vertikalen Lageänderungen bedingten Druckschwankungen (hydrostatische Druckänderungen) im Wasserkissen besondere Anforderungen an die Druckregelung gestellt.

Aus der **DE-A-35 36 073** ist eine Vorrichtung zum Konstanthalten des Anpreßdruckes des Wasserkissens eines Lithotripters bekannt. Über einen Druckregler wird ein ringförmiger Hohlkörper, der an der Innenwand des Lithotripters anliegt, durch Druckluft aufgeblasen. Dadurch wird Flüssigkeit im Inneren des Lithotripters verdrängt, sodaß sich der Anpressdruck erhöht. Wo der Druckaufnehmer angeordnet ist, ist nicht offenbart.

Die **DE-A-35 03 477** beschreibt einen Wasserkreislauf mit einem Wasserkissen für ein Ultraschalldiagnosegerät, mit einem Kreislauf zum Erwärmen des Wassers, zum Befüllen und Entleeren des Wasserkissens und mit Mitteln zum Einstellen des Druckes im Wasserkissen. Es enthält auch eine Entlüftungsvorrichtung für das Wasserkissen. Das Problem, den Wasserdruck auch dann richtig einzustellen, wenn das Gesamtgerät gekippt ist, stellt sich hier nicht.

In der nachveröffentlichten **EP-A-0 265 741** ist ein Wasserkreislauf eines Geräts zur Stosswellenbehandlung, insbesondere eines Lithotripters, beschrieben. Das Gerät enthält einen Hauptkreislauf zum Temperieren des Wassers, zum Befüllen und Entleeren eines Wasserkissens und zur präzisen Regelung des Druckes im Wasserkissen, einen Spülkreislauf zum Entlüffen des Wasserkissens und einen Drucksensor, dessen Messwert zur Steuerung der Befüllung und Entleerung des Wasserkissens verwendet wird. Ein paralleles, gleichzeitiges Arbeiten beider Kreisläufe ist nicht vorgesehen. Zur Regelung des Ankoppeldrucks sind keine besonderen Lehren gegeben.

Aus der **EP-A-0 090 138** ist ein Lithotripter bekannt, bei dem der Patient in einer oben offenen Wanne gelagert ist. Es ist ein Wasserkreislauf vorgesehen zum Befüllen und Entfernen der Gasblasen. Da kein Ankoppelkissen vorgesehen ist, ist auch keine Regelung des Druckes an der Einkoppelstelle der Stosswellen in den Körper notwendig.

Aufgabe der Erfindung ist es, einen Wasserkreislauf vorzuschlagen, der folgende Anforderungen erfüllt:
- Ankoppelung des Wasserkissens an den Patientenkörper in jeder beliebigen Lage zwischen der Stosswellenquelle und dem zweiten Fokus
- Anpassung an die verschieden korpulenten Patienten
- Blasenfreie Ankoppelung des Wasserkissens an den Patientenkörper im Einwirkungsbereich der Stosswelle
- Einstellung eines vorgewählten Ankoppelungsdrucks des Wasserkissens an den Patientenkörper
- Konstanthaltung des Ankoppelungsdrucks im Arbeitsbereich der Therapieeinheit (verschiedene Arbeitsniveaus und Ankoppelrichtungen: von oben, von unten und von der Seite)
- Schneller Druckausgleich bei gleichzeitig grosser Befüll- und Entleerungsgeschwindigkeit des Wasserkissens bei möglichst geringem Überschwingen des Niveaus in bestimmten Grenzen.

Diese Aufgabe wird erfindungsgemaß gelöst von einem Wasserkreislauf mit den Merkmalen des Hauptanspruchs.
Ausführungen der Erfindung sind Gegenstände von Unteransprüchen.

Die Funktion des erfindungsgemäßen Wasserkreislaufs beruht auf dem Zusammenwirken eines Spül- und eines Hauptkreislaufs. Beide Kreisläufe werden vorteilhaft mit einer Pumpe gemeinsam betrieben. Bei Schwankungen der Flüssigkeitsströme werden die Druckpotentiale mit Druckhalteventilen konstant gehalten. Sämtliche Funktionen des Wasserkreislaufs können durch geeignete Überwachungseinrichtungen kontrolliert werden.

Die Erfindung lässt sich bei verschiedenen Wasserkissen-Typen anwenden: sowohl verschiedene Grössen oder Bauarten können mit dem erfindungsgemässen Wasserkreislauf bedient werden. So können Wasserkissen mit flexiblem Faltenbalg und Folienabschluss oder Wasserkissen aus gespannten Folien bedient werden, wie zum Beispiel Silikonfolien, die im Ankoppelbereich eine ballonförmige Ausweitung haben.
Die parallelen Haupt- und Spülkreisläufe erlauben ein schnelles Anfahren und Abfahren des Wasserkissens an den Patientenkörper für den Ankoppelungs- und Abkoppelungsvorgang, da eine hohe Befüll- und Entleerungsgeschwindigkeit möglich ist. Die Erfindung ermöglicht einen schnellen Druckausgleich und geringe Überschwingweiten des Niveaus im Wasserkissen. Da aufgrund des parallelen Systemaufbaus bei Störungen nur begrenzte Überdrücke im Wasserkissen entstehen können, ist eine hohe Patientensicherheit gegenüber Überdrücken bei Störungen gewährleistet.

Da ein Drucksensor in unmittelbarer Nähe der Ankoppelfläche des Wasserkissens angeordnet ist, stellt sich automatisch der richtige Ankoppeldruck auf jedem Niveau und in jeder Lage des Wasserkissens ein (Ankoppelung des Wasserkissens an den Patientenkörper von oben, von unten und von der Seite möglich) . Dies erlaubt eine individuelle, sensible Einstellbarkeit des Ankoppelungsdruckes.

In einer bevorzugten Ausführung wird der Drucksensor als Differenzdruckaufnehmer ausgebildet und erlaubt die Kompensation von atmosphärischen Luftdruckschwankungen und eine sehr genaue Einstellung des Ankoppelungsdrucks im Wasserkissen an dem Patientenkörper (der Druck des Koppelmediums im Wasserkissen ist im Millibarbereich genau regelbar).

Die Ausführung mit offenem Ausgleichsbehälter erlaubt eine einfache Befüllung der Anlage und eine problemlose Entlüftung des Wasserkissens.

Der erfindungsgemässe Wasserkreislauf kann um eine Entgasungsanlage erweitert werden, die dann dem beschriebenen Wasserkreislauf parallel geschaltet werden kann. Damit können die im Koppelmedium gelösten Gase und Gase, die zum Beispiel an der Funkenstrecke durch Kavitation entstehen, leicht aus dem Gesamtsystem entfernt werden.

In einer weiteren Ausführung können Strömungswächter als einfache, aber sichere Überwachungsgeräte für die Funktion des Kreislaufes eingesetzt werden.

Die Erfindung wird anhand einer Figur näher erläutert.

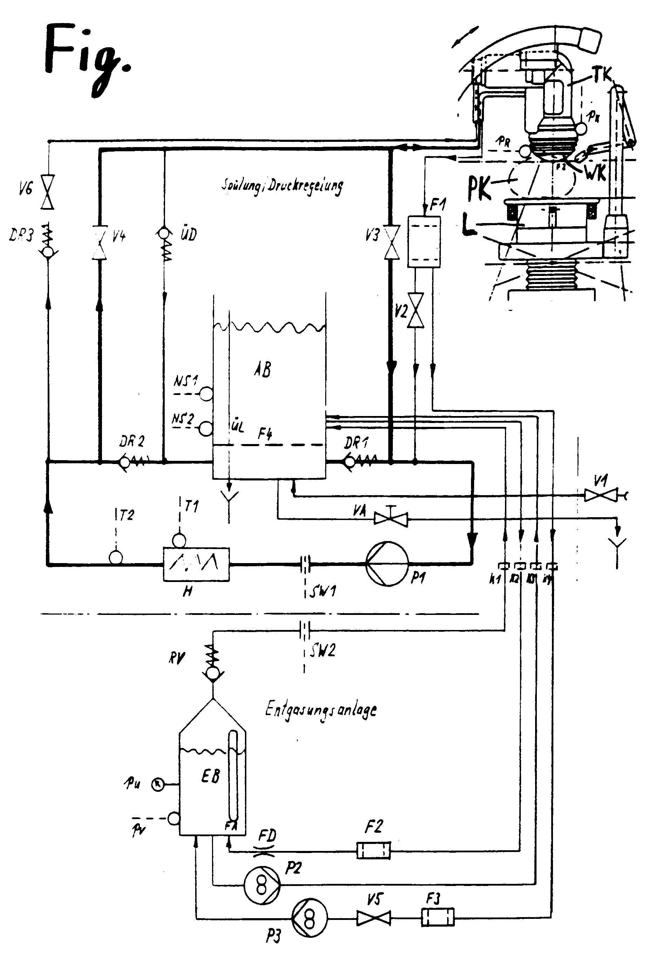

Die Figur zeigt rechts oben eine Lithotripter mit der Liege L, dem Patientenkörper PK und dem an einer Schiene geführten Therapiekopf TK, der eine Stosswellenquelle und - hier darunter angeordnet - ein Wasserkissen WK zum Einkoppeln der Stosswellen in den Patientenkörper PK besitzt.
Der erfindungsgemässe Wasserkreislauf besteht im wesentlichen aus den vier Komponenten:
- Hauptkreislauf
- Spülkreislauf
- Druckregelung und
- Entgasungsanlage,
die nachfolgend näher beschrieben sind.

### Hauptkreislauf (dick gezeichnet)

Das Spül- und Druckregelungssystem wird mit der Umwälzpumpe P1 betrieben. Mit dieser Pumpe wird im Hauptkreislauf das Wasser kontinuierlich umgewälzt und mit der Heizung H auf 37°C temperiert. Der Strömungsweg des Hauptkreislaufes ist wie folgt:
- Pumpe P1
- Strömungswächter SW1
- Heizung H, mit den Temperatursensoren T1 für Übertemperatur und T2 für die Temperaturregelung
- Druckhalteventil DR2
- Ausgleichsbehälter AB
- Druckhalteventil DR1
- Pumpe P1.

Die Druckhalteventile DR1 und DR2 erzeugen für die Druckregelung auf der Saug- und Druckseite der Umwälzpumpe P1 definierte Druckpotentiale, die nahezu unabhängig von Durchflußschwankungen sind. Die Kennlinien dieser Druckhalteventile verlaufen derart, dass Durchflußschwankungen des durchfliessenden Mediums nur eine geringe Druckschwankung zur Folge haben.
Diese Durchflußschwankungen entstehen, wenn im Hauptkreislauf durch das Umschalten der Ventile V3 oder V4, die für die Druckregelung zur Verfügung stehen, das Befüllen oder Entleeren des Wasserkissens WK (siehe Druckregelung weiter unten) ausgelöst wird.

Durch das Umschalten der Ventile durch die Druckregelung wird jeweils ein Teilstrom des Koppelmediums in den Parallelkreislauf hinein- oder herausgeführt, wodurch die Strömungsverhältnisse im Hauptkreislauf verändert werden. Zum Befüllen des Wasserkissens WK wird ein bestimmter Überdruck, zum Entleeren ein bestimmter Unterdruck, benötigt. Die Druckhalteventile erzeugen nun für den Zulauf zum Wasserkissen WK (Ventil V4) einen nahezu konstanten Überdruck und am Ablauf vom Wasserkissen (Ventil V3) auf der Saugseite der Umwälzpumpe P1 einen nahezu konstanten Unterdruck Dieser Über- und Unterdruck ist zusätzlich für den Betrieb des Spülkreislaufs (weiter unten erläutert) erforderlich. Die erfindungsgemäss beschriebene Druckkonstanthaltung auf der Druck- und Saugseite der Umwälzpumpe P1 ist für ein rasches Befüllen und Entleeren des Wasserkreislaufes mit der Druckregelung notwendig.
Im Ausgleichsbehälter AB werden die Flüssigkeitsvolumenschwankungen im Gesamtkreislauf kompensiert, die durch die Druckregelung des Wasserkissens WK und durch das Ankoppeln und Abkoppeln des Wasserkissens WK an den Patientenkörper entstehen. Der Ausgleichsbehälter AB dient zusätzlich der selbsttätigen Entlüftung des Systems.

### Druckregelung:

Der Druck im Wasserkissen WK wird mit einem elektronischen Drucksensor PR detektiert und dessen Ausgangssignal über eine Auswerteelektronik an den Druckregler, zum Beispiel einen Dreipunkt-Schrittregler, geführt.
Der Drucksensor PR ist bevorzugt auf dem Niveau der Ankoppelfläche des Wasserkissens WK installiert und mit einer kurzen Verbindungsleitung mit dem Innenteil des Wasserkissens WK verbunden. Die Position des Drucksensors PR gegenüber den Drehachsen des Therapiekopfes TK ist so gewählt, dass nahezu im gesamten Arbeitsbereich des Therapiekopfes TK ein mittlerer Wasserdruck in unmittelbarer Nähe der Ankoppelfläche des Wasserkissens WK gemessen wird. Mit dieser Verfahrensweise lässt sich der Ankoppelungsdruck des Wasserkissens WK an den Patientenkörper PK genau messen und somit auch genau regeln.
Die Druckmeßzelle des Druckaufnehmers PR ist bevorzugt als Differenzdruckaufnehmer ausgeführt, das heisst es wird der Druckunterschied zwischen dem Druck im Wasserkissen WK und dem Umgebungsdruck (atmosphärischer Luftdruck) gemessen. Atmosphärische Luftdruckschwankungen werden hierdurch kompensiert.
Mit dem Druckregler werden in Abhängigkeit von der Grösse und dem Vorzeichen der Regelabweichung (Differenz zwischen Ist- und Sollwert) das Ventil V3 oder V4 mit einer von der Regelabweichung abhängigen Frequenz angesteuert. Mit dem Ventil V4 wird das Wasserkissen WK gefüllt und mit dem Ventil V3 entleert, wobei sich analog hierzu der Druck im Wasserkissen WK erhöht oder erniedrigt. Wenn keine Regelabweichung vorhanden ist, sind beide Ventile V3 und V4 geschlossen.
Mit diesem Druckregelungssystem wird eine gute Anpassung an das Zeitverhalten der Regelstrecke (Wasserkissen WK) bei optimaler Störgrössenausregelung (Druckschwankungen) erreicht.

### Spülkreislauf (dünn gezeichnet) :

Mit dem Spülkreislauf wird das Wasserkissen entlüftet, Schmutzpartikel werden von der Stosswellenquelle ausgefiltert und erwärmtes und entgastes Wasser zum Wasserkissen WK geführt.
Der Spülkreislauf ist dem Hauptkreislauf parallel geschaltet. Der Strömungsweg führt vom Hauptkreislauf ausgehend über das Druckhalteventil DR3 und das Absperrventil Spülung V6 zum Wasserkissen WK und über den Filter F1 und das Absperrventil Spülkreislauf V2 zum Hauptkreislauf zurück. Das Druckhalteventil DR3 erzeugt im Zusammenhang mit den Druckhalteventilen DR1 und DR2 des Hauptkreislaufes nahezu konstante Druckverhältnissefür den Spülkreislauf, so dass beim Betrieb der Druckregelung die Strömung im Spülkreislauf oder im Hauptkreislauf nicht abreissen kann. Mit DR3 wird zusätzlich der Zu- und Ablauf des Spülkreislaufs bezüglich des Wasserkissens WK abgeglichen, damit sich der Spülkreislauf im Gleichgewicht befindet und keine ständige Befüllung oder Entleerung des Wasserkissens WK erfolgt. Bei bestimmten Wasserkissentypen (eingespannte Folien) wird auf regelungstechnischen Gründen das Strömungsgleichgewicht im Spülkreislauf mit Hilfe eines Strömungsregelungssystems abgeglichen. Das Ventil V6 ist dann durch ein Proportionalventil ersetzt.

### Entgasungsanlage:

Die Entgasungsanlage besteht im wesentlichen aus dem Entgasungsbehälter EB und den mit Pumpen P2, P3, Ventilen V5, RV und Filtern F2, F3 versehenen Leitungen, die über Kupplungsverbindungen K1, K2, K3, K4 mit dem Rest der Anlage verbindbar sind. Die Entgasungsanlage hat im wesentlichen zwei Betriebsphasen:
1. Befüllphase
2. Entgasungsphase.

### Befüllphase:

Während der Befüllphase wird das Wasser über den Filter F1 vom Spülkreislauf abgesaugt. Im Filter F1 vorhandene Luft, die sich in diesem nach der Neubefüllung des Systems oder nach dem Elektrodenwechsel ansammelt, wird in der Befüllphase der Entgasungsanlage abgeführt. Die Entgasungspumpe P2 ist ausgeschaltet, die Befüllpumpe P3 eingeschaltet und das Ventil V5 geöffnet. Der Entgasungsbehälter EB wird über den Siebfilter F3 und das Ventil V5 mit der Befüllpumpe P3 gefüllt. Das Rückschlagventil RV öffnet und der Entgasungsbehälter EB wird über den Ausgleichsbehälter AB entlüftet. Mit dem Strömungswächter SW2 wird die vollständige Befüllung des Entgasungsbehälters EB erfasst und mit einer einstellbaren Zeitverzögerung in den Entgasungsmodus umgeschaltet.

### Entgasungsphase

Während der Entgasungsphase sind Ventil V5 und das Rückschlagventil RV geschlossen. Der Strömungsweg führt vom Ausgleichsbehälter AB über den Siebfilter F2 und die Festdrossel FD zum Entgasungsbehälter EB. Mit der Entgasungspumpe P2 wird das Wasser abgepumpt und durch die Drosselwirkung von der Festdrossel FD (Venturirohr) eine weitere Druckabsenkung erzeugt, die durch die Verschiebung des Lösungsgleichgewichtes die Entgasung des Wasser bewirkt. Um Druckverluste zwischen der Entgasungspumpe P2 und dem Entgasungsbehälter EB möglichst gering zu halten, ist die Entgasungspumpe P2 direkt am Gehäuse des Entgasungsbehälters EB angeflanscht. Von der Pumpe P2 führt der Strömungsweg zurück zum Ausgleichsbehälter AB.
Der Unterdruck im Entgasungsbehälter EB liegt beispielsweise bei ca. -0.85 bar gegenüber dem atmosphärischen Luftdruck. Durch den Entgasungsbehälter EB wird die Verweilzeit des Wassers im Vakuum wesentlich erhöht. Das hier vorliegende Wasser/Luftblasengemisch trennt sich durch das Aufsteigen der Luftblasen. Die aufsteigenden Luftblasen wirken wiederum als Entgasungskeime, die den Entgasungsprozess verstärken. Durch eine rauhe Oberfläche des Entgasungsbehälters EB wird der Prozess nach dem gleichen Prinzip zusätzlich verstärkt. Nach der Trennung des Wasser/Luftblasengemisches sammelt sich die vom Wasser abgeschiedene Luft oberhalb des Wasserspiegels im Entgasungsbehälters EB. Der Wasserspiegel senkt sich hierdurch kontinuierlich ab. Nach einem festgelegten Zeitraum, zum Beispiel fünf Minuten, bei dem sich das Niveau im Entgasungsbehälter EB um einen bestimmten Betrag absenkt, wird das System mit einem Zeitrelais wieder in die Befüllphase geschaltet. Die Befüll- und Entgasungsphase wechseln sich fortwährend ab.

Die in der Figur verwendeten Abkürzungen werden nachstehend nochmals im Überblick angegeben:
- AB =: Ausgleichsbehälter: Spülung/Druckregelung
- EB =: Entgasungsbehälter
- ÜL =: Überlauf
- H =: Heizung
- T1 =: Temperatur-Sensor: Übertemperatur
- T2 =: Temperatur-Sensor: Temperatur-Regelung
- SW1 =: Strömungswächter: Hauptkreislauf
- SW2 =: Strömungswächter: Entgasungsanlage
- NS1 =: Niveau-Sensor: automatische Nachbefüllung
- NS2 =: Niveau-Sensor: Störmeldung Füllstand Ausgleichsbehälter
- PR =: Drucksensor Druckregelung Wasserkissen
- PK =: Druckschalter Kollisionsschutz Ellipsoid
- PV =: Vakuumschalter Entgasungsanlage
- PN =: Unterdruckanzeige
- F1 =: Filter Hauptkreislauf
- F2 =: Siebfilter Entgasung
- F3 =: Siebfilter Entgasung
- F4 =: Filtermatte Ausgleichsbehälter
- P1 =: Umwälzpumpe Hauptkreislauf
- P2 =: Entgasungspumpe
- P3 =: Befüllpumpe Entgasungsbehälter
- VA =: Absperrventil Ablauf
- V1 =: Ventil Zulauf
- V2 =: Absperrventil Spülkreislauf
- V3 =: Ventil Druckregelung
- V4 =: Ventil Druckregelung
- V5 =: Steuerventil Entgasung
- V6 =: Absperrventil Spülung
- RV =: Rückschlagventil Entgasung
- DR1 =: Druckhalteventil
- DR2 =: Druckhalteventil
- DR3 =: Druckhalteventil
- ÜD =: Überdruckventil
- ÜL =: Überlauf
- FA =: Füllstandsanzeige Entgasungsbehälter
- FD =: Festdrossel Entgasung
- K1 =: Kupplungsverbindung
- K2 =: Kupplungsverbindung
- K3 =: Kupplungsverbindung
- K4 =: Kupplungsverbindung

## Patentansprüche

1. Wasserkreislauf eines Geräts zur Stosswellenbehandlung, insbesondere eines Lithotripters, mit einem Hauptkreislauf zum Erwärmen des Wassers, zum Befüllen und Entleeren eines Wasserkissens (**WK**) und zur präzisen Regelung des Druckes im Wasserkissen, einem zum Hauptkreislauf parallelen Spülkreislauf zum Entlüften des Wasserkissens und einem Drucksensor (**PR**), dessen Messwert zur Steuerung der Befüllung und Entleerung des Wasserkissen (**WK**) verwendet wird, wobei der Drucksensor (**PR**) in unmittelbarer Nähe der Ankoppelfläche des Wasserkissens (**WK**) angeordnet ist.

2. Wasserkreislauf nach Anspruch 1, **dadurch gekennzeichne**t, dass im Hauptkreislauf folgende Elemente aufeinanderfolgen:
- Pumpe (**P1**)
- Heizung (**H**)
- mit Ventil (**V4**) geregelter Zulauf zum Wasserkissen (**WK**)
- Druckhalteventil (**DR2**)
- Ausgleichsbehälter (**AB**)
- Druckhalteventil (**DR1**)
- mit Ventil (**V3**) geregelter Ablauf vom Wasserkissen (**WK**)
- Pumpe (**P1**).

3. Wasserkreislauf nach Anspruch 1 oder Anspruch 2, **dadurch gekennzeichnet**, dass im Spülkreislauf folgende Elemente aufeinanderfolgen:
- Pumpe (**P1**)
- Heizung (**H**)
- Druckhalteventil (**DR3**)
- Absperrventil Spülung (**V6**)
- Wasserkissen (**WK**)
- Filter (**F1**)
- Absperrventil Spülkreislauf (**V2**)
- Pumpe (**P1**).

4. Wasserkreislauf nach mindestens einem der vorhergehenden Ansprüche, **dadurch gekennzeichnet**, dass der Drucksensor (**PR**) ein Differenzdruckaufnehmer ist, der mit der Umgebungsluft in Verbindung steht.

5. Wasserkreislauf nach einem der Ansprüche 2 bis 4, **dadurch gekennzeichnet**, dass der Ausgleichsbehälter (**AB**) offen ist, eine Filtermatte (**F4**) und Niveau-Sensoren (**NS1, NS2**) enthält.

6. Wasserkreislauf nach einem der vorhergehenden Ansprüche, **gekennzeichnet durch** eine Entgasungsanlage.

7. Wasserkreislauf nach einem der vorhergehenden Ansprüche, **gekennzeichnet durch** Strömungswächter (**SW1, SW2**) in einem oder mehreren der Kreisläufe.

## Claims

1. Water circuit of an apparatus for shock-wave treatment, in particular of a lithotripter, having a main circuit for heating the water, for filling and emptying a water cushion (WK) and for precise regulation of the pressure in the water cushion, a flushing circuit running parallel with the main circuit for ventilating the water cushion, and a pressure sensor (PR), the measuring value of which is used to control the filling and emptying of the water cushion (WK), in which respect the pressure sensor (PR) is arranged in the immediate vicinity of the coupling surface of the water cushion (WK).

2. Water circuit according to claim 1, **characterised in that** the following elements are arranged in the main circuit in the following order:
- pump (P1)
- heater (H)
- valve-controlled (V4) delivery to the water cushion (WK)
- pressure-holding valve (DR2)
- compensating vessel (AB)
- pressure-holding valve (DR1)
- valve-controlled (V3) drain from the water cushion (WK)
- pump (P1).

3. Water circuit according to claim 1 or claim 2, **characterised in that** the following elements are arranged in the flushing circuit in the following order:
- pump (P1)
- heater (H)
- pressure-holding valve (DR3)
- shut-off valve flushing (V6)
- water cushion (WK)
- filter (F1)
- shut-off valve flushing circuit (V2)
- Pump (P1).

4. Water circuit according to at least one of the above claims, **characterised in that** the pressure sensor (PR) is a differential-pressure pickup which is connected to environmental air.

5. Water circuit according to one of claims 2 to 4, **characterised in that** the compensating vessel (AB) is open and contains a filter mat (F4) and level sensors (NS1, NS2).

6. Water circuit according to one of the above claims, **characterised by** a degassing device.

7. Water circuit according to one of the above claims, **characterised by** flow monitors (SW1, SW2) in one or a plurality of circuits.

## Revendications

1. Circuit de circulation d'eau d'un appareil de traitement par ondes de choc, en particulier d'un lithriteur, comportant un circuit principal pour réchauffer l'eau, remplir et vider un coussin à eau (WK) et pour réguler avec précision la pression à l'intérieur du coussin, un circuit de vidage en parallèle avec le circuit principal pour dégazer le coussin à eau et un capteur de pression (PR) dont la grandeur de mesure est utilisée pour commander le remplissage et la vidange du coussin à eau (WK), le capteur de pression (PR) étant disposé à proximité immédiate de la surface de couplage du coussin à eau (WK).

2. Circuit selon la revendication 1, caractérisé par le fait que le circuit principal comprend successivement les éléments suivants:
- pompe (P1)
- chauffage (H)
- arrivée d'eau au coussin à eau (WK) régulée par valve (V4)
- soupape de retenue (DR2)
- réservoir tampon (AB)
- soupape de retenue (DR1)
- sortie du coussin à eau (WK) régulée par valve (V3)
- pompe (P1)

3. Circuit selon la revendication 1 ou 2, caractérisé par le fait que le circuit de vidage comprend successivement les éléments suivants:
- pompe (P1)
- chauffage (H)
- soupape de retenue (DR3)
- soupage d'arrêt (V6)
- coussin à eau (WK)
- filtre (F1)
- soupape d'arrêt circuit de vidange (V2)
- pompe (P1)

4. Circuit selon l'une au mois des revendications précédentes, caractérisé par le fait que le capteur de pression (PR) est un capteur de pression différentiel qui est relié à l'atmosphère.

5. Circuit selon l'une des revendications 2 à 4, caractérisé par le fait que le réservoir tampon (AB) est ouvert et contient un tissus filtrant (F4) et des détecteurs de niveau (NS1, NS2).

6. Circuit selon l'une des revendications précédentes, caractérisé par une installation de dégazage.

7. Circuit selon l'une au moins des revendications précédentes, caractérisé par des détecteurs d'écoulement (SW1, SW2) dans un ou plusieurs des circuits.
